(19) Europäisches Patentamt
European Patent Office
Office européen des brevets

(11) Publication number: **0 500 641 B1**

## EUROPEAN PATENT SPECIFICATION

(45) Date of publication of patent specification: **08.03.95**

(51) Int. Cl.[6]: **C07K 14/435**, C07K 16/18, A61K 39/395, A61K 38/02, C12P 21/08

(21) Application number: **90916498.0**

(22) Date of filing: **13.11.90**

(86) International application number: **PCT/GB90/01742**

(87) International publication number: **WO 91/07434 (30.05.91 91/12)**

(54) **CONTROL OF MILK SECRETION.**

(30) Priority: **13.11.89 GB 8925594**

(43) Date of publication of application:
**02.09.92 Bulletin 92/36**

(45) Publication of the grant of the patent:
**08.03.95 Bulletin 95/10**

(84) Designated Contracting States:
**BE CH DE ES FR GR LI NL**

(56) References cited:

**Chemical Abstracts, vol. 110, no. 17, 24 April 1989, (Columbus, Ohio, US), A. Prentice et al., page 494, abstract 151872b.**

**Chemical Abstracts, vol. 106, no. 13, 30 March 1987, (Columbus, Ohio, US), C.J. Wilde et al., page 464, abstract 100147d**

**Chemical Abstracts, vol.100, no. 21, 21 May 1984, (Columbus, Ohio US), A.J. Henderson et al., page 424, abstract 172338r**

(73) Proprietor: **BRITISH TECHNOLOGY GROUP LTD**
**101 Newington Causeway**
**London SE1 6BU (GB)**

(72) Inventor: **ADDEY, Caroline, Victoria, Pauline**
**11 Chalmers Road**
**Ayr KA7 2RO (GB)**
Inventor: **PEAKER, Malcolm**
**13 Upper Crofts**
**Alloway**
**Ayr KA7 4OX (GB)**
Inventor: **WILDE, Colin, James**
**23 Raithhill**
**Alloway**
**Ayr KA7 4UF (GB)**

(74) Representative: **Percy, Richard Keith**
**Patents Department**
**British Technology Group Ltd**
**101 Newington Causeway**
**London SE1 6BU (GB)**

## Description

This invention relates to a new protein isolated from goat's milk and the use of the protein or antibodies thereto for the control of milk secretion in lactating animals.

### Description of the prior art

The rate of milk secretion by a lactating animal is regulated by the frequency of milk removal. In other words, there is a mechanism which acts to match the animal's supply of milk to the demand of her offspring or of a farmer's milking regime. Part of this control is achieved by the release of galactopoietic hormones during suckling or milking. However, studies by workers at the Hannah Research Institute, Ayr, Scotland on lactating goats have shown that another factor is involved. This is an inhibitor which decreases milk secretion at a local level, i.e. at the individual gland of an udder.

It has already been shown that the inhibitor is present in a goat milk fraction containing whey proteins of molecular weight 10-30 KDa, this range of molecular weights being determined by the nominal mesh sizes of filters used in ultrafiltration of the whey. The effect has been demonstrated both in vitro and in vivo. The in vitro technique, described by C.J. Wilde et al., Biochem. J. 242, 285-288 (1987), consists in culturing explanted pieces of rabbit mammary tissue with and without the milk fraction and demonstrating the inhibition of lactose and casein synthesis. See also G.M. Stewart et al., J. Endocrinology 118, R1-R3 (1988). In the in vivo technique, C.J. Wilde et al., Quarterly Journal of Experimental Physiology 73, 391-397 (1988), the mily fraction was injected into a single mammary gland of goats via the teat canal. A temporary dose-dependent reduction of milk yield, specific to that gland, was observed. Other papers describing various other aspects of this research are C.J. Wilde et al., Biochem. Soc. Trans. 15, 916-917 (1988), C.J. Wilde et al., Biochem. Biophys. Acta., 92, 315-319 (1989), J. McKinnon et al., J. Endocrinol. 119 (supplement), 167 (1988) and M. Peaker and D.R. Blatchford, J. Dairy Res., 55, 41-48.

A brief report of a lecture given by Dr. C.J. Wilde to the International Society for Research in Human Milk and Lactation in New Orleans in March 1989 was published in the Mammary Gland Biology Newsletter (May 1989). The report said that the inhibitor had been purified and its structure determined, but no details were given and the author of the report has since admitted that it was pure speculation without factual foundation.

It has therefore remained a problem to determine whether the goat milk inhibitor is a single compound or two or more compounds acting in concert, to isolate it or them from the 10-30 KDa fraction, and to purify it sufficiently for identification, with a view to chemical or biological synthesis.

### Summary of the invention

It has now been found possible to separate the 10-30 KDa fraction by anion exchange chromatography into a number of peaks and it has been determined that the inhibitor activity is concentrated mainly in a particular peak which is not one of the most abundant. A protein has been isolated from this peak and its properties determined, and its activity as an inhibitor of milk secretion confirmed. Further, antibodies to the inhibitor have been found at least partly to neutralise the effect of the inhibitor.

Conventional ion-exchange chromatography using DEAE (diethylaminoethyl)-cellulose ion-exchange resin did not resolve the constituents of the 10-30KDa fraction effectively. As a consequence, bioassay experiments on material eluted from this column gave equivocal results. Similarly, gel filtration of the 10-30KDa fraction did not separate it effectively into individual components.

The protein of the invention inhibits milk secretion by lactating goats and has a molecular weight, as determined by gel filtration chromatography of the protein in its glycosylated form of about 7.6 KDa. It is the major protein present in the third significant peak when a nominally 10-30kDa fraction of the whey proteins of the milk is resolved on an anion exchange column containing particles of monodisperse hydrophilic polymers having pendant $-CH_2N(CH_3)_3^+$ groups, the particle diameter being $10 \pm 0.5\mu$m, especially a "Mono Q" column, using 20 mM bis tris propane (1,3-bis[tris(hydroxymethyl)methylamino]propane) buffer, pH 7.0 and a 0 to 1.0 M sodium acetate gradient. This definition (1) can optionally be supplemented. The supplementary definition (2) provides that it is the major protein present in the second significant peak obtained when the said third peak of definition (1) is resolved on a chromatofocussing column containing particles of monodisperse hydrophilic polymers having pendant tertiary ($-N^+HR_2$) and quaternary ($-N^+R_3$) amine groups, the Rs being organic substituents (not necessarily the same), the particle diameter being $10 \pm 0.5\mu$m, especially a "Mono P" column, using 0.025 M piperazine-HCl, pH 5.5 and amphoteric buffer of pH 4.0, to create a pH gradient in the range 5.5-4.5.

A property useful for defining the protein is the isoelectric point (pI). It has been found that the third peak in definition (1) gives a pI of about 4.8 to 4.9 in isoelectric focussing in a tube of polyacrylamide gel. However, the second peak in definition (2) was eluted on chromatofocussing at an apparent pH 4.2.

The invention includes the inhibitor protein in glycosylated or unglycosylated form.

Antibodies to the protein, whether polyclonal, monoclonal or engineered, are within the scope of this invention.

The use of the inhibitor to decrease milk yield or an antibody thereto to suppress at least partly the action of the inhibitor, to goats or other animals is within the invention.

Brief description of the drawings

Figures 1 and 2 show the resolution of the 10-30 KDa fraction by an ion-exchange chromatography in two different buffers;

Figure 3 shows the results of an ELISA in which samples from various peaks of the Figure 1 chromatography are tested for ability to bind to antiserum raised against the peak containing the protein of the invention; and

Figure 4 shows the resolution by chromatofocussing of three protein peaks obtained by anion exchange chromatography.

Description of the preferred embodiments

The protein of the invention exists in milk in glycosylated form. It is believed that the effect of glycosylation is simply for attachment of the protein to the appropriate cells within the mammary gland. It would be expected, therefore, that the protein could be administered locally to the gland in an unglycosylated form.

Using the 10-30 KDa fraction, it has been demonstrated that the inhibition of lactose and casein synthesis in mammary explant culture is dependent on the dose of the inhibitor-containing fraction. Further, when the explants have been exposed to the inhibitor-containing fraction, washed and re-cultured in fresh medium in the absence of the inhibitor, the capacity to synthesise lactose and casein is recovered. In vivo, it is found that administration of the protein to the mammary gland causes the milk yield to decrease within hours, with full recovery of yield 24-36h after a single administration. However when a change in milking frequency - and therefore autocrine control - was sustained over weeks, there was an effect on the synthetic capacity i.e. degree of differentiation of the secretory cells attributable to the autocrine inhibitor. These long-term effects on mammary cell activity are accompanied by changes in the number of cell-surface hormone receptors for prolactin. Thrice-daily milking of lactating goats for 4 weeks increases cell differentiation and prolactin receptor number per cell, whereas a decrease in milking efficiency extending over 21 weeks reduces secretory cell differentiation and prolactin receptor number. Therefore, these long-term effects, and also the acute regulation by the autocrine inhibitor of the invention could be due primarily to modulation of the sensitivity of individual glands to endocrine control.

Antibodies can be raised against the protein of the invention by any conventional methods, e.g. as polyclonal antisera, mouse monoclonal antibodies, or engineered antibodies made by recombinant techniques, by any of the currently available methods. Passive immunisation methods can then be used to generate a reduction in the effect of the natural inhibitor, when this is desired in order to increase milk yield. Frequently, however, there will be a need to reduce milk yield in order to meet milk quotas, in which event the inhibitor itself is administered. Conventional carriers and adjuvants known in vaccination can be used.

The invention is applicable to any animal responsive to the inhibitor defined herein. Since the 10-30 KDa goat milk fraction has been successfully found to reduce milk accumulation and relevant enzyme activities when injected into the mammary gland of rabbits, it is likely that the inhibitor will be effective in some other lactating animals.

A significant effect on goat milk yield was obtained by intraductal injection of an inhibitor fraction produced from 100ml of milk. A significant unilateral effect on milk accumulation in rabbits was obtained by injecting 4 glands on one side each with 1.0-1.25ml of 20 times concentration 10-30KDa fraction i.e. from 20-25ml of milk. At an estimated inhibitor concentration in milk of 0.1$\mu$g/ml, this suggests that an effective intraductal dose in goats is 10mg/gland. Effects can therefore be expected from injection in the range 1 to 50$\mu$g especially 5 to 20$\mu$g, of inhibitor.

This dose would be repeated as required, e.g. daily, and possibly reduced when given over long periods.

The protein of the invention can be obtained from goat's milk by the method described in Example 1 or some variant thereon. It can be recovered in pure form from an eluate by extensive dialysis against water (using an appropriate membrane for retention of the protein, e.g. with a nominal molecular weight cut-off of about 6 KDa) and freeze-drying. However, it is expected that it would be synthesised by protein synthesis or by a recombinant DNA method.

The following Examples illustrate the invention.

EXAMPLE 1

This Example describes the preparation and properties of the inhibitor of the invention.

1. Preparation of goat milk fractions

Milk was obtained at the morning milking from British Saanen goats in mid-lactation (except where indicated), and was defatted by centrifugation (2500 g, 15°C, 20 min) and filtered through glass wool. Defatted milk was dialysed against 40 volumes of 10 mM Hepes, pH 7.4, using Spectropor-1 dialysis membrane (molecular weight cut-off 6000-8000 Daltons), or centrifuged (80,000 g, 15°C, 2h), yielding a pellet of casein micelles and a clear supernatant containing whey proteins.

Portions of the whey protein fraction were subjected to ultrafiltration using filters with nominal cut-off values of molecular weight 10,000, 30,000, 50,000 and 300,000 Daltons (Da). In each case, the retentate volume was decreased by 95% and washed with 4 volumes of 10 mM Hepes, pH 7.4. The filtrate containing material of m.w. less than 10,000 Da was concentrated by freeze-drying. Other filtrates, and the retentate obtained with the 30,000 Da filter, were concentrated by ultrafiltration with a 10,000 Da filter. Fractions were sterilized by gamma-irradiation or by filter sterilization. The 10,000-30,000 Da fraction was dialysed exhaustively against distilled water and concentrated by freeze-drying for anion exchange chromatography.

2. Anion exchange chromatography of goat whey proteins

The 10-30 KDa whey fraction was resolved on a "MonoQ HR 10/10" anion exchange column (Pharmacia) using FPLC (Fast Protein Liquid Chromatography). "Mono Q" is a strong anion exchanger based on Mono Beads - monodisperse hydrophilic polymer particles (10±0.5$\mu$m diamter) which bind negatively-charged components through quaternary amine groups [-CH$_2$N(CH$_3$)$_3$$^+$]. Two buffer systems were used:

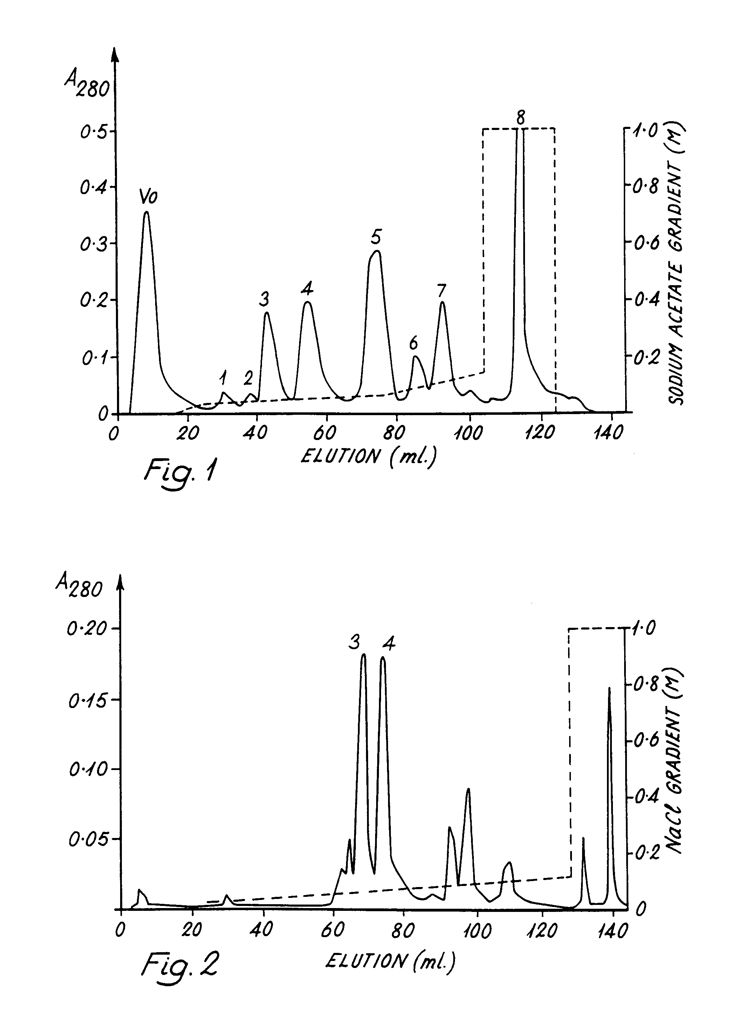

(a) 20 mM bis tris propane (1,3-bis [tris(hydroxymethyl) methylamino]propane), pH 7.0, using a sodium acetate gradient to elute individual proteins; this buffer system was used to prepare proteins for bioassay experiments (Figure 1).

(b) 10 mM imidazole, pH 7.0, using an elution gradient of sodium chloride (Figure 2); this buffer system produced a similar separation, but with sharper peaks than (a).

The whey fraction was dissolved in the appropriate starting buffer (20 mM bis tris propane or 10 mM imidazole) at twice its concentration in the original milk and solutions were adjusted to pH 7.0. Before chromatography, the sample and buffers were filtered through 0.2 $\mu$m filters. In addition, buffers were degassed before use. 2 ml of the 2 x concentrated whey fraction was loaded for each separation; the flow rate was 4.0 ml/min.

Fractions containing protein peaks eluted from the column were dialysed extensively against distilled water, freeze-dried and stored at -20°C, before use in the next stage.

Figure 1 of the drawings shows the elution of protein from the chromatography column. Protein concentration, as absorption of light at 280 nm, on the left-hand ordinate is plotted by a solid line against cumulative volume of eluted material on the abscissa. The right-hand ordinate is calibrated to show the sodium acetate gradient, from 0 to 1.0M, used in the eluant (a), and the gradient is plotted by a broken line. The peaks are labelled Vo = void volume containing material not bound to the column and then 1-8 in order of elution.

Figure 2 of the Drawings is a similar plot to Figure 1, but for the imidazole buffer system, the right-hand ordinate being calibrated in 0-1.0 M sodium chloride gradient. The two central peaks at elution volumes of 65-80 ml. are believed to correspond to Nos. 3 and 4, respectively, of Figure 1.

Figures 1 and 2 relate to typical chromatographies, but the relative sizes of the peaks vary from one preparation to another.

### 3. Mammary explant bioassay of goat milk fractions

Mammary tissue was cultured as explants, small pieces of parenchymal tissue approximately 1cm$^3$ and weighing 0.5-0.7 mg. Explants were prepared from mammary tissue of mid-pregnant New Zealand White rabbits as described by R. Dils & I.A. Forsyth in Methods in Enzymology 72, 724-742 (1981). The explants were cultured in a defined culture medium (Medium 199; Gibco Europe Ltd., Paisley, UK) on stainless steel grids each holding 30 explants, so that the explants were in contact with the medium but not completely submerged in it. The medium was supplemented throughout with insulin (5$\mu$g/ml), cortisol (100ng/ml) and prolactin (1$\mu$g/ml). Explants were cultured in this medium under an atmosphere of air/$CO_2$ (19:1 v/v) for 42 h, with replenishment of medium after 24 h. At this time, groups of explants (3 or 4 groups per treatment) were transferred into fresh medium containing hormones and one of the fractions of goat milk obtained by anion exchange chromatography as described above. The milk fractions were dissolved in 10 mM Hepes, pH 7.4, at twice their concentration in the original milk, and added to an equal volume of two times concentrated culture medium, so as to be at 100% of their original milk concentration in normal strength culture medium. Control cultures, containing only the diluent for the milk fractions, were included in each experiment. Average rates of lactose and casein synthesis during a further 6 h culture in the presence or absence of milk fraction were measured by the addition of [U-$^{14}$C]glucose (U = uniformly labelled; 0.18 mCi/mmol) and L-[4.5-$^3$H]leucine (2.22mCi/mmol) respectively to this culture medium. At the end of the 6 h period, explants and culture medium were separated and stored frozen in liquid nitrogen.

Explants were homogenized at 4°C in 1.0 ml of 10mM Tris/ HCl, pH 7.0. containing 5mM ethyleneglycol-bis-(2-aminoethyl ether) N,N,N',N'-tetraacetic acid (EGTA) and 2mM phenylmethanesulphonyl fluoride by 10 strokes with a glass/PTFE homogenizer, followed by sonication for 30s (Kontes ultrasonic cell disruptor, 30% maximum power), and a particle-free supernatant was prepared by centrifugation at 10.000g for 5 min. [$^3$H]-labelled casein was prepared from the particle-free supernatant by precipitation at its isoelectric point, and the precipitate was subjected to SDS-polyacrylamide gel electrophoresis, as described by C.J. Wilde et al., Exp. Cell Res. 151, 519-532 (1984). Bands corresponding to casein polypeptides were visualized by staining with Coomassie brilliant blue, and were excised and counted for [$^3$H] radioactivity as described by S.M. Russell et al., Biochim. Biophys. Acta 714, 34-45 (1982). [$^{14}$C] lactose was selectively precipitated from explant homogenates and culture medium using ethanol/diethyl ether (3:1, v/v), N.J. Kuhn & A. White, Biochem. J. 148, 77-84 (1975) and the radioactivity of the precipitate counted. Results were corrected for carry-through of [$^{14}$C] glucose from culture medium (usually < 0.08%), by measuring [$^{14}$C] radioactivity after extraction of uncultured medium. The addition of milk fractions did not affect the distribution of secreted products between the extracellular space of the explants and the medium.

The amount of radioactive material (casein and lactose) was expressed as a percentage of that produced by the explants to which no milk fraction had been added. The results are shown in Table 1. Numbers of determinations are shown in parenthesis.

Table 1

| Fraction | Lactose synthesis % inhibition | Casein synthesis % inhibition |
|---|---|---|
| Unfractionated | 29.5 ± 5.6 (13)* | 32.2 ± 5.2** |
| Void volume | 6.2 ± 7.7 (9) | 7.9 ± 8.7 |
| Peaks 1 + 2 | 1.0 ± 15.6 (5) | 13.1 ± 9.6 |
| Peak 3 | 31.5 ± 7.2 (13)* | 35.1 ± 3.6*** |
| Peak 4 | 30.1 ± 7.7 (7) | 7.2 ± 10.2 |
| Peak 5 | 10.8 ± 20.6 (11) | 17.7 ± 7.6 |
| Peak 6 | 5.5 ± 5.2 (10) | 6.3 ± 8.3 |
| Peak 7 | 14.2 ± 8.4 (5) | 1.0 ± 8.0 |
| Peak 8 | 23.4 ± 10.5 (3) | (13.9 ± 7.9§) |

§ Stimulation, i.e. casein synthesis apparently exceeded that of the control in which no milk was added. Peak 8 contained a lot of protein (not resolved by the gradient) which, in the 3 experiments in which it was tested, may have had non-specific effects on the explants.

*$p < 0.05$;
**$p < 0.01$;
***$p < 0.001$

From Table 1 it will be seen that peaks 3 and 4 were the most active in inhibiting lactose synthesis. Lactose synthesis is a major determinant of milk yield.

## 4. Gel filtration chromatography of peak 3

Gel filtration of peak 3 was carried out using an "FPLC" chromatography system and a Superose 12 HR 10/30 column (Pharmacia). The buffer was 50 mM Tris/HCl, pH 7.5 containing 100 mM KCl, which was filtered (0.2 $\mu$m filter) and degassed before use. Samples (routinely 1-10 $\mu$g in a maximum volume of 200 $\mu$l) were dissolved in the same buffer and filtered before use (0.2 $\mu$m filter). The column was calibrated using molecular weight standards in the m.w. range 200,000-12,400 (Sigma MW-GF-200 kit) and also aprotinin (molecular weight 6,500) and bovine $\alpha$-lactalbumin (molecular weight 14,200). Calibration curves of log[molecular weight] versus $V_e/V_o$ were prepared, where $V_o$ = void volume and $V_e$ = elution volume of each protein. $V_o$ was determined using Dextran Blue (Sigma; approximate molecular weight 2,000 KDa). The molecular weight of peak 3 protein was thus determined to be about 7.6 KDa. (An attempt at m.w. determination by SDS-PAGE gave anomalous results: it appears that high molecular weight aggregates form). The unexpectedly low molecular weight can probably be explained by clogging of the nominally 10,000 Dalton filter during ultrafiltration, allowing smaller molecules to be retained.

## 5. Assessment of Protein Glycosylation

Hexose determination of the peak 3 protein separated by gel filtration chromatography was by the "Anthrone" method as described by R.G. Spiro, Methods in Enzymology, 8, 4-5 (1966). By this method, peak protein contained 70 $\mu$g hexose per 100 $\mu$g protein) (average of 2 determinations), indicating glycosylation. Control unglycosylated proteins (bovine $\alpha$-lactalbumin and RNase A) contained negligible amounts of hexose under the assay conditions.

## 6. Isoelectric focussing of goat whey proteins

Isoelectric focussing was performed in tube gels (diameter, 4mm; length 11.5 cm). 4% polyacrylamide gels were prepared essentially as described by P.H. O'Farrell J. Biol. Chem. 250, 4007-4021 (1975) using a mixture of ampholines (4% v/v pH range 5-8; 1% v/v pH range 3.5-10; BioRad), which gave a linear gradient in the range 4.0-9.0. Samples (25 $\mu$g of the peak 3 protein) were dissolved in a solution containing 9.5M urea, 2% (w/v) NP40, 1.6% (v/v) pH 5-8 ampholines and 0.4% (v/v) pH 3.5-10 ampholines. The anodic and cathodic solutions were 10 mM $H_3PO_4$ and 20 mM NaOH respectively. Electrophoresis was at 300 V for 18 h, followed by 400 V for 4 h. Gels were extruded and fixed first in 25% (v/v) isopropanol/10% (v/v) acetic acid, then in 5%. (w/v) TCA/5% (w/v) sulphosalicylic acid/1% (v/v) methanol, and were stained 25% (v/v) isopropanol/10% (v/v) acetic acid containing 0.1% (w/v) Coomassie Blue. Destaining was in isopropanol/acetic acid.

The isoelectric point of the peak 3 protein was thus found to be 4.85.

## 7. Amino acids

Amino acid composition was determined and the empirical ratio of amino acids in the peak 3 was calculated using Arginine = 2 as the standard value. Results are shown in Table 2.

Table 2

| Amino acid | Net amount (nmoles) | Amino acid ratio |
|---|---|---|
| Asx (Asp or Asn) | 6.937 | 6 |
| Thr | 2.587 | 2 |
| Ser | 5.439 | 4 |
| Glx (Gln or Glu) | 9.173 | 7 |
| Pro | 4.404 | 4 |
| Gly | 8.181 | 7 |
| Ala | 6.204 | 5 |
| Val | ≫standard, amount unknown | 1 |
| Ile | 1.398 | 2 |
| Leu | 4.604 | 4 |
| Tyr | 0.752 | 1 |
| Phe | 2.178 | 2 |
| Lys | 3.762 | 3 |
| His | 1.138 | 1 |
| Arg | 2.466 | 2 |
| Met | 5.747 | 5 |
| Cys | 1.398 | 1 |
| Trp | - | 0 |

The molecular weight calculated from the above empirical amino acid composition data was 7136. This is consistent with the m.w. 7600 obtained by gel permeation chromatography of the glycosylated protein, the difference being accountable for by glycosylation.

N-terminal amino acid sequencing of the first 10 amino acids gave SEQ ID NO: 1 in which the unknown fifth amino acid is possibly Val and the eighth amino acid is possibly Glu.

## 8. Catalysis of Lactose Synthesis

Peak 3 does not promote the synthesis of lactose by galactosyltransferase. This indicates that it is not related to α-lactalbumin, the principal whey protein. Lactose synthetase activity is associated with peaks 5 to 7, indicating that these peaks contain the several forms of α-lactalbumin in goal milk.

## 9. Other properties of peak 3

(a) Hydrophilicity

Reversed phase chromatography of peak 3 indicated that it is strongly hydrophilic.

(b) Spectral Analysis

The peak 3 protein absorbs maximally at 261 nm. The calculated molar extinction coefficient for absorption at 280 nm is $5.12 \times 10^8$. This absorbance is high compared with other whey proteins, which indicates that peak 3 constitutes only a small proportion of the 10-30 KDa fraction.

EP 0 500 641 B1

(c) Stability

The peak 3 protein, purified by gel filtration chromatography, was stored for 2 weeks as a lyophilized powder at -20°C, or in solution in 10 mM Hepes buffer pH 7.0 at 37°C. When re-analysed by the same gel filtration chromatography technique, there was no evidence of either a decrease in protein content of the peak, or the appearance of low molecular degradation products. Therefore, the protein appears to be stable in the conditions under which it has been prepared and tested.

10. Separation of peak 3 components by chromatofocussing

Chromatofocussing separates proteins on the basis of their isoelectric point (pI). Resolution with the Pharmacia "Mono P HR 5/20" column is such that molecules differing in pI by only 0.02 pH units can be separated "Mono P" is a weak anion exchanger, based on mono beads, i.e. monodisperse hydrophilic polymer particles ($10 \pm 0.5\mu m$ diameter) into which various tertiary ($-N^+HR_2$) and quaternary ($-N^+R_3$) amine groups are introduced. "Mono P" has a buffering capacity and the amount of charge it carries will vary with pH. Consequently, its ionic capacity will also vary with pH. In chromatofocussing, a pH gradient is formed on the column by equilibrating it with start buffer and eluting with another buffer which is added in increasing amounts, thereby adjusting the solution progressively to a lower pH. Proteins bound to the column at the starting pH are eluted at different points on the pH gradient according to their pI.

A "Mono P" column was first equilibrated with 0.025M piperazine-HCl pH 5.5, and a pH gradient (5.5-4.0) was formed in situ in the column by elution with "Polybuffer 74", pH 4.0 (diluted 1/10 in distilled water). "Polybuffer" (Pharmacia) contains numerous amphoteric buffering substances of different pKa. Buffers were filtered through $0.2\mu m$ filters and degassed before use. The flow rate was 0.75 ml/min. Samples (peak 2, 3, & 4 from the anion exchange column) were dissolved at approximately 50-100 $\mu g$ in 1.0 ml of 0.025M piperazine-HCl pH 5.5 and filtered through $0.2\mu M$ filters. Fractions containing protein peaks were collected, dialysed extensively against distilled water, freeze-dried and stored at -20°C.

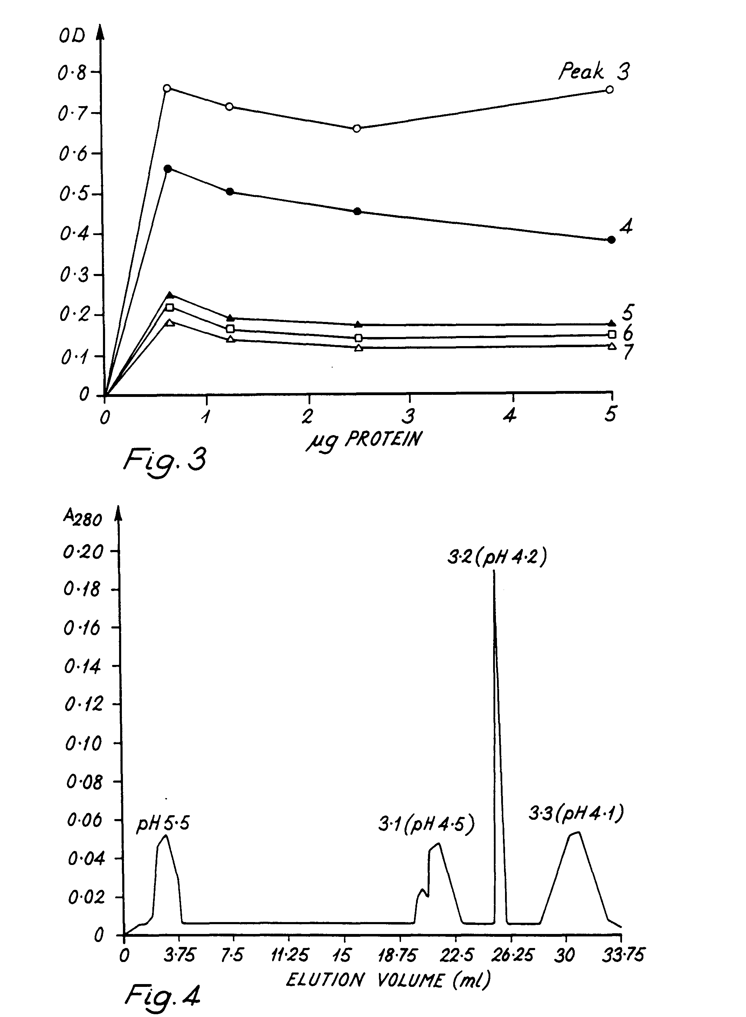

Figure 4 of the drawings shows the elution profile obtained using peak 3. Protein concentration, as absorption of light at 280 nm, on the left hand ordinate is plotted by a solid line against cumulative volume of eluted material on the abscissa. Peak 3 contained three major components label led 3.1, 3.2 and 3.3. The apparent pHs were estimated using Whatman indicator papers (type CS, pH 3.8-5.5) and may not reflect accurate pI values. Each peak did however elute consistently at the same cumulative volume. Figure 4 shows a typical elution profile, but the relative sizes of the peaks may vary from one preparation to another.

Anion exchange peaks 2 and 4 each contained three components, designated 2.1, 2.2, 2.3 and 4.1, 4.2, 4.3. Comparison of all three profiles suggested that 3.1 arises by contamination with 2.1; 3.2, the major component of peak 3, is also a "contaminant" of peak 4 (i.e. 4.2); 3.3 may arise through contamination with 4.3, the major constituent of peak 4. Peaks 2.2 and 2.3 elute at positions which do not correspond to 3.2 or 3.3

11. Mammary explant bioassay of peak 3.2

The procedure of Section 3 was repeated on the chromatofocussed fractions from peak 3, except that they were added to bioassay culture medium to give a final concentration of three times their concentration in the original milk. The results are shown in Table 3. Numbers of determinations are shown in parenthesis.

Table 3

| Fraction | Lactose synthesis % inhibition | Casein synthesis % inhibition |
|---|---|---|
| 3.1 | 4.5 ± 30.1 (4) | 6.0 ± 9.8 |
| 3.2 | 19.6 ± 16.9 (4) | 32.2 ± 12.6 |
| 3.3 | (13.7 ± 28.8 (4)§) | (12.5 ± 8.70 §) |
| § Stimulation, i.e. lactose or casein synthesis apparently exceeded that of the control in which no milk was added. | | |

The results of Table 3 suggest that inhibitory activity is most consistently associated with the major peak 3.2.

## 12. Gel Filtration of chromatofocussed peaks

Section 4 was repeated on the fractions 3.1, 3.2 and 3.3, the chromatofocussed peaks, obtained in Section 10. They all eluted at a similar position implying molecular weights of about 7600 Da.

## EXAMPLE 2

This Example illustrates the preparation of antibodies to the inhibitor and their use in detecting the inhibitor and their ability to block the inhibition of the synthesis of lactose and casein.

To prepare rabbit anti-(goat peak 3 protein) for use in an ELISA, the peak 3 was dissolved in 0.5 ml of phosphate-buffered saline, pH 7.6, and administered as an emulsion with Complete Freund's adjuvant. Female New Zealand White Rabbits were given a primary subcutaneous injection of 100 $\mu$g protein at multiple sites along the back. 28 days later, a second subcutaneous injection was given as above but using Incomplete Freund's adjuvant. Rabbits were bled 7 and 14 days later from the marginal ear vein, by making a small cut with a sterile scalpel blade.

ELISA plates (Flow Laboratories) were coated with 1-5 $\mu$g of individual peaks 3, 4, 5, 6 and 7 prepared from the 10-30 KDa whey fraction, each dissolved in 100 $\mu$l of phosphate-buffered saline (PBS). After incubation overnight at 4°C, the plates were washed 3 times with PBS and 0.1% "Tween 20". 150 $\mu$l of PBS, 0.1% "Tween 20" and 5% BSA were added to each well and the plates were stood for 1 h at room temperature, to allow saturation of non specific binding sites. Plates were washed 3 times as above and 100 $\mu$l of the rabbit anti-(goat peak 3 protein) antiserum (diluted 1:200 in PBS) was added to each well. After 2 h at 40°C, the plates were again washed and 100 $\mu$l of peroxidase-linked anti-rabbit IgG antiserum (Scottish Antibody Production Unit) diluted 1:1000 in PBS + 0.1% "Tween 20" + 0.5% BSA were added. The plates were incubated again for 2 h at 4°C. They were then washed 5 times as above and 100 $\mu$l of ortho-phenylene diamine (OPD) substrate (0.4 mg/ml OPD in 11.38 mM $Na_2HPO_4$ and 46.45 mM citric acid pH 6.0 containing 0.01% $H_2O_2$) was added to each well. Colour was allowed to develop in the dark for 20 min, and the reaction was terminated by the addition of 50 $\mu$l 4M $H_2SO_4$. Absorbances were read at 492 nm using a Multiscan microtitre reader (Flow). Antibody only and antigen only control values were subtracted from each test reading.

The results are shown graphically in Fig. 3 in which units of optical density are plotted on the ordinate against amounts of the peak material on the abscissa. Each value represents the average of two determinations. It will be seen that the antibody binds strongly to the peak 3 material, as expected but that there is some cross-reactivity with peak 4.

Results of two bioassay experiments showed that inhibition of lactose and casein synthesis by the inhibitor was partially reversed when the above anti serum was included (at an arbitrary concentration) in the culture medium. Lactose synthesis was inhibited by 19% when no antibody was added, but by 9% with the antibody present. Casein synthesis inhibition was inhibited by 39% when no antibody was added, but by 21% when it was present. Rabbit control serum had no effect on the inhibition of lactose synthesis, and only slightly reversed the inhibition of casein synthesis.

## Claims
## Claims for the following Contracting States : BE, CH, LI, DE, FR, NL

1. The isolated protein, in glycosylated or unglycosylated form, which inhibits milk secretion by lactating goats and which is the major protein present in the material of the third significant peak when a nominally 10-30 KDa fraction of the whey proteins of the milk is resolved on a anion exchange column containing particles of monodisperse hydrophilic polymers having pendant $-CH_2N(CH_3)_3$ groups, the particle diameter being 10±0.5$\mu$m, using 20 mM bis tris propane buffer, pH 7.0 and a 0 to 1.0 M sodium acetate gradient, and which protein also possesses a molecular weight, as determined by gel filtration chromatography of material from said third peak of about 7.6 KDa and an isoelectric point, as determined by isoelectric focussing of material from said third peak in a tube of polyacrylamide gel, of about 4.8 to 4.9.

2. The protein according to claim 1 which is further defined as the major protein present in the second significant peak obtained when said third peak of claim 1 is resolved on a chromatofocussing column containing particles of monodisperse hydrophilic polymers having pendant tertiary ($-N^+HR_2$) and quaternary ($-N^+R_3$) amine groups, the Rs being organic substituents, the particle diameter being 10 ± 0.5$\mu$M, using 0.025M piperazine-HCl, pH 5.5 and amphoteric buffer of pH 4.0 to create a pH gradient of

EP 0 500 641 B1

5.5-4.5.

3. Antibody to the protein according to claim 1 or 2.

4. Use of the protein according to claim 1 or 2, for regulating the lactation of animals.

5. Use of antibody to the protein according to claim 1 or 2, for regulating the lactation of animals.

**Claims for the following Contracting States : ES, GR**

1. A process of preparing a protein by protein synthesis or a recombinant DNA method, and optionally raising an antibody against said protein, characterised in that one prepares an isolated protein, in glycosylated or unglycosylated form, which inhibits milk secretion by lactating goats and which is the major protein present in the material of the third significant peak when a nominally 10-30 KDa fraction of the whey proteins of the milk is resolved on a anion exchange column containing particles of monodisperse hydrophilic polymers having pendant $-CH_2N(CH_3)_3^+$ groups, the particle diameter being $10\pm0.5\mu$m, using 20 mM bis tris propane buffer, pH 7.0 and a 0 to 1.0 M sodium acetate gradient, and \,oM which protein also possesses a molecular weight, as determined by gel filtration chromatography of material from said third peak of about 7.6 KDa and an isoelectric point, as determined by isoelectric focussing of material from said third peak in a tube of polyacrylamide gel, of about 4.8 to 4.9.

2. A process according to claim 1, characterised in that one prepares the protein defined in claim 1 and further defined as the major protein present in the second significant peak obtained when said third peak of claim 1 is resolved on a chromatofocussing column containing particles of monodisperse hydrophilic polymers having pendant tertiary ($-N^+HR_2$) and quaternary ($-N^+R_3$) amine groups, the Rs being organit substituents, the particle diameter being $10 \pm 0.5\mu$M, using 0.025M piperazine-HCl, pH 5.5 and amphoteric buffer of pH 4.0 to create a pH gradient of 5.5-4.5.

**Patentansprüche**
**Patentansprüche für folgende Vertragsstaaten : BE, CH, LI, DE, FR, NL**

1. Isoliertes Protein in glycosilierter oder nichtglycosilierter Form, welches die Milchsekretion von milchgebenden Ziegen inhibiert, und welches als Hauptprotein im Material des dritten wesentlichen Peaks vorliegt, wenn eine nominelle 10 bis 30 KDa Fraktion der Molkeproteine der Milch auf einer Anionenaustauschersäule, die Teilchen eines monodispergierten hydrophilen Polymers mit freien $-CH_2N(CH_3)_3$-Gruppen enthält, wobei der Teilchendurchmesser $10 \pm 0,5 \mu$m ist, unter Verwendung eines 20 mM Bistrispropanpuffers, pH 7,0, und eines Natriumacetatgradienten von 0 bis 1,0 M aufgetrennt wird, und dieses Protein auch ein Molekulargewicht, bestimmt durch Gelfiltrationschromatographie des Materials aus dem dritten Peak, von ca. 7,6 KDa und einem isoelektrischen Punkt, bestimmt durch isoelektrische Fokussierung des Materials aus dem dritten Peak in einem Polyacrylamidgelröhrchen, von ca. 4,8 bis 4,9 besitzt.

2. Protein nach Anspruch 1, welches ferner definiert wird als das im zweiten wesentlichen Peak enthaltene Hauptprotein, das erhalten wird, wenn der dritte Peak aus Anspruch 1 auf einer chromatofokussierenden Säule, welche Teilchen eines monodispergierten hydrophilen Polymers mit freien tertiären ($-N^+HR_2$) und quaternären ($-N^+R_3$) Amingruppen enthält, wobei die Rs organische Substituenten sind und der Teilchendurchmesser $10 \pm 0,5 \mu$m ist, unter Verwendung von 0,025M Piperazin-HCl, pH 5,5, und einem amphoteren Puffer von pH 4,0 unter Erzeugung eines pH-Gradienten von 5,5 bis 4,5 aufgetrennt wird.

3. Antikörper gegen das Protein nach Anspruch 1 oder 2,.

4. Verwendung des Proteins nach Anspruch 1 oder 2 zur Regulierung der Milchproduktion von Tieren.

5. Verwendung des Antikörpers gegen das Protein nach Anspruch 1 oder 2 zur Regulierung der Milchproduktion von Tieren.

10

**Patentansprüche für folgende Vertragsstaaten : ES, GR**

1.  Verfahren zur Herstellung eines Proteins durch proteinische Synthese oder Verfahren der Rekombinanten DNA und gegbenfalls die Richtung eines Antikörpers gegen das Protein, dadurch gekennzeichnet daß ein isoliertes Protein in glycosilierter oder nichtglycosilierter Form, welches die Milchsekretion von milchgebenden Ziegen inhibiert, und welches als Hauptprotein im Material des dritten wesentlichen Peaks vorliegt, wenn eine nominelle 10 bis 30 Kda Fraktion der Molkeproteine der Milch auf einer Anionenaustauschersäule, die Teilchen eines monodispergierten hydrophilen Polymers mit freien -$CH_2N(CH_3)_3$-Gruppen enthält, wobei der Teilchendurchmesser 10 ± 0,5 $\mu$m ist, unter Verwendung eines 20 mM Bistrispropanpuffers, pH 7,0, und eines Natriumacetatgradienten von 0 bis 1,0 M aufgetrennt wird, und dieses Protein auch ein Molekulargewicht, bestimmt durch Gelfiltrationschromatographie des Materials aus dem dritten Peak, von ca. 7,6 Kda und einem isoelektrischen Punkt, bestimmt durch isoelektrische Fokussierung des Materials aus dem dritten Peak in einem Polyacrylamidgelröhrchen, von ca. 4,8 bis 4,9 besitzt, hergestellt wird.

2.  Verfahren nach Anspruch 1, dadurch gekennzichnet daß ein Protein, welches in Anspruch 1 definiert wird und welches ferner definiert wird als das im zweiten wesentlichen Peak enthaltene Hauptprotein, das erhalten wird, wenn der dritte Peak aus Anspruch 1 auf einter chromatofokussierenden Säule, welche Teilchen eines monodispergierten hydrophilen Polymers mit freien tertiären (-$N^+HR_2$) und quaternären (-$N^+R_3$) Amingruppen enthält, wobei die Rs organische Substituenten sind und der Teilchendurchmesser 10 ± 0,5 $\mu$m ist, unter Verwendung von 0,025 M Piperasin-HCl, pH 5,5, und einem amphoteren Puffer von pH 4,0 unter Erzeugung eines pH-Gradienten von 5,5 bis 4,5 aufgetrennt wird, hergestellt wird.

**Revendications**
**Revendications pour les Etats contractants suivants : BE, CH, LI, DE, FR, NL**

1.  Protéine isolée, sous forme glycosylée ou non glycosylée, qui inhibe la sécrétion de lait des chèvres allaitantes et qui est la protéine majeure présente dans le matériel du troisième pic important lorsqu'une fraction nominalement de 10-30 kDa des protéines du lactosérum du lait est séparée sur une colonne échangeuse d'anions contenant des particules de polymères hydrophiles monodispersés ayant des groupes latéraux -$CH_2N(CH_3)_3$, le diamètre des particules étant de 10 ± 0,5 $\mu$m, en utilisant du tampon bis-tris propane 20 mM à pH 7,0 et un gradient d acétate de sodium de 0 à 1,0 M laquelle proteine a egalement un poids moléculaire, déterminé par chromatographie par filtration sur gel du matériel dudit troisième pic, d'environ 7,6 kDa et un point isoélectrique, déterminé par isoélectrofocalisation du matériel dudit troisième pic dans un tube de gel de polyacrylamide, d'environ 4,8 à 4,9

2.  Protéine selon la revendication 1 qui est de plus définie comme la protéine majeure présente dans le second pic important obtenu lorsqu'on sépare ledit troisième pic de la revendication 1 sur une colonne de chromatofocalisation contenant des particules de polymères hydrophiles monodispersés ayant des groupes latéraux amine tertiaire (-$N^+HR_2$) et quaternaire (-$N^+R_3$), les R étant des substituants organiques, le diamètre des particules étant de 10 ± 0,5 $\mu$m, en utilisant de la pipérazine-HCl 0,025 M à pH 5,5 et un tampon amphotère de pH 4,0 pour créer un gradient de pH de 5,5 à 4,5.

3.  Anticorps contre la protéine de la revendication 1 ou 2.

4.  Utilisation de la protéine de la revendication 1 ou 2 pour la régulation de la lactation des animaux.

5.  Utilisation de l'anticorps contre la protéine de la revendication 1 ou 2 pour la régulation de la lactation des animaux.

**Revendications pour les Etats contractants suivants : ES, GR**

1.  Un procédé de préparation d'une protéine par synthése protéinique ou un procédé de l'ADN recombiné et eventuellement l'enlevant d'un anticorps contre ladite protéine, caractérisé en ce qu'on prépare une protéine isolée, sous forme glycosylée, qui inhibe la sécrétion de lait des chèvres allaitantes et qui est la protéine majeure présente dans le matériel du troisième pic important lorsqu'une fraction nominalement de 10-30 kDa des protéines du lactosérum du lait est séparée sur une colonne échangeuse d'anions contenant des particules de polyméres hydrophiles monodispersés ayant des groupes latéraux -CH$_2$N(CH$_3$)$_3$, le diamétre des particules étant de 10 ± 0,5 $\mu$m, en utilisant du tampon bis-tris propane 20 mM à pH 7,0 et un gradient d'acétate de sodium de 0 à 1,0 M, laquelle protéine a également un poids moléculaire, déterminé par chromatographie par filtration sur gel du matériel dudit troisième pic, d'environ 7,6 kDa et un point isoélectrique, déterminé par isoélectrofocalisation du matériel dudit troisiéme pic dans un tube de gel de polyacrylamide, d'environ 4,8 à 4,9.

2. Un procédé selon la revendication 1 caracterisé en ce qu'on prépare la protéine défincé dans la revendication 1 et qui est de plus définie comme la protéine majeure présente dans le second pic important obtenu lorsqu'on on sépare ledit troisième pic de la revendication 1 sur une colonne de chromatofocalisation contenant des particules de polyméres hydrophiles monodispersés ayant des groupes latéraux amine tertiaire (-N$^+$HR$_2$) et quaternaire (-N$^+$R$_3$), les R étant des substituants organiques, le diamètre des particules étant de 10 ± 0,5 $\mu$m, en utilisant de la pipérazine-HCl 0,025 M à pH 5,5 et un tampon amphotére de pH 4,0 pour créer un gradient de pH 5,5 à 4,5.

Fig. 1

Fig. 2

Fig. 3

Fig. 4